## Europäisches Patentamt

## European Patent Office

## Office européen des brevets

(19)

(11) Veröffentlichungsnummer: **0 098 966**

**A2**

(12) # EUROPÄISCHE PATENTANMELDUNG

(21) Anmeldenummer: 83105568.6

(22) Anmeldetag: 07.06.83

(51) Int. Cl.³: **G 01 N 1/10**

(30) Priorität: 12.06.82 DE 3222234

(43) Veröffentlichungstag der Anmeldung:
25.01.84 Patentblatt 84/4

(84) Benannte Vertragsstaaten:
AT BE CH DE FR IT LI NL

(71) Anmelder: Alfons Schwarte GmbH
Theodor-Schwarte-Strasse 61
D-4730 Ahlen i.W.(DE)

(72) Erfinder: Eismann, Josef, Ing. grad.
Schiege 6
D-4730 Ahlen 5(DE)

(72) Erfinder: Helmig, Reinhold, Ing. grad.
Beethovenstrasse 15
D-4780 Lippstadt 4(DE)

(72) Erfinder: Gromes, Manfred
Julius-Abeler-Strasse 17
D-4730 Ahlen(DE)

(74) Vertreter: Busse & Busse Patentanwälte
Postfach 1226 Grosshandelsring 6
D-4500 Osnabrück(DE)

(54) **Vorrichtung zur Entnahme von Milchproben.**

(57) Die Vorrichtung dient zur Entnahme von Milchproben aus einer bei der Übernahme von Milch in einen Milchsammelbehälter aus der Übernahmeleitung einer Übernahmevorrichtung abgezweigten, in einem Zwischenbehälter angesammelten Teilmilchmenge. Der Zwischenbehälter ist dabei über eine Zulaufleitung und über eine Rücklaufleitung an die Übernahmeleitung angeschlossen sowie mit einer ventilgesteuerten Probenabfüllvorrichtung versehen, unter deren Auslauf Probebehälter bereitstellbar sind. Zur Schaffung einer besonders einfachen, leicht in zuverlässiger Weise bedienbaren Vorrichtung, die sowohl an den Saugbereich, als auch an den Druckbereich der Übernahmeleitung von Übernahmevorrichtungen anschließbar ist und bei stark herabgesetzten Verschleppungsgefahren in besonders hohem Maße repräsentativ ist, umfaßt die Probenabfüllvorrichtung ein in die Rücklaufleitung eingeschaltetes Ventilgehäuse und darin einen Ventilkörper, der in einer ersten Stellung einen Durchlaß durch das Ventilgehäuse und damit die Rücklaufleitung sperrt, in einer zweiten Stellung einen Durchlaß im Ventilgehäuse für einen dieses durchspülenden Milchrücklauf durch die Rücklaufleitung öffnet und in einer dritten Stellung zusätzlich einen Probemilchauslauf in den bereitgestellten Probebehälter freigibt.

./...

Fig.1

0098966

- 1 -

Vorrichtung zur Entnahme von Milchproben          DB/Dr

Die Erfindung bezieht sich auf eine Vorrichtung zur Entnahme von Milchproben aus einer bei der Übernahme von Milch in einen Milchsammelbehälter aus der Übernahmeleitung einer Übernahmevorrichtung abgezweigten, in einem Zwischenbehälter angesammelten Teilmilchmenge gemäß dem Oberbegriff des Anspruchs 1.

Vorrichtungen dieser Art sind in zahlreichen Ausführungen bekannt, die jedoch für die Entnahme einer verschleppungsarmen, repräsentativen Probe einen hohen baulichen und schaltungstechnischen Aufwand verwirklichen.

Der Erfindung liegt die Aufgabe zugrunde, eine besonders einfache, leicht in zuverlässiger Weise bedienbare Vorrichtung der eingangs genannten Art zu schaffen, die sowohl an den Saugbereich, als auch an den Druckbereich der Übernahmeleitung von Übernahmevorrichtungen anschließbar ist und bei stark herabgesetzten Verschleppungsgefahren in besonders hohem Maße repräsentativ ist.

Die Erfindung löst diese Aufgabe durch die Merkmale des Anspruchs 1. Hinsichtlich bedeutsamer weiterer Ausgestaltungen wird auf die Ansprüche 2 bis 12 verwiesen.

Nachfolgend werden mehrere Ausführungsbeispiele des Gegenstands der Erfindung in Verbindung mit der Zeichnung beschrieben. In der Zeichnung zeigen:

Fig. 1        eine teilweise abgebrochene, schematische Gesamtansicht einer Vorrichtung zur Entnahme von
              Milchproben in einer ersten Ausführung nach der
              Erfindung,

Fig. 2        eine Ansicht ähnlich Fig. 1 einer in ihrem An-
              schluß an die Übernahmeleitung abgewandelten
              zweiten Ausführung,

Fig. 3        einen Querschnitt durch die Probenabfüllvorrich-
bis 5         tung in Fig. 1 und 2 in drei verschiedenen Betriebsstellungen,

Fig. 6        eine Ansicht ähnlich Fig. 1 einer dritten Ausführung der Vorrichtung zur Entnahme von Milchproben nach der Erfindung,

Fig. 7        eine Ansicht ähnlich Fig. 6 einer vierten, hinsichtlich ihres Anschlusses an die Übernahme-
              leitung abgeänderten Ausführung der Vorrichtung
              nach Fig. 6, und

Fig. 8        Querschnittsdarstellungen ähnlich Fig. 3 bis 5
bis 10        der Probenabfüllvorrichtung in Fig. 6 und 7.

Die in der Zeichnung dargestellten Ausführungsformen der
erfindungsgemäßen Vorrichtung zur Entnahme von Milchproben
umfassen einen Zwischenbehälter 1 zur Aufnahme einer Teilmilchmenge, die bei der Übernahme von Milch aus beispielsweise Hofbehältern od. dgl. in einen Milchsammelbehälter,
insbesondere einen Milchsammelwagen, aus der Übernahmeleitung 2 einer nur bruchstückhaft wiedergegebenen Übernahmevorrichtung fortlaufend abgezweigt wird. Die Übernahmevorrichtung umfaßt bekannter- und üblicherweise eine in die
Übernahmeleitung 2 eingeschaltete, selbst-ansaugende Pumpe 3,
einen nicht dargestellten, vor- oder hinter der Pumpe 3
in die Übernahmeleitung 2 eingeschalteten Luftabscheider
sowie einen in Strömungsrichtung 4 der zu übernehmenden

0098966

- 3 -

Milch dem Luftabscheider nachgeordneten Durchlaufzähler bzw. ein ähnliches Mengenmeßgerät.

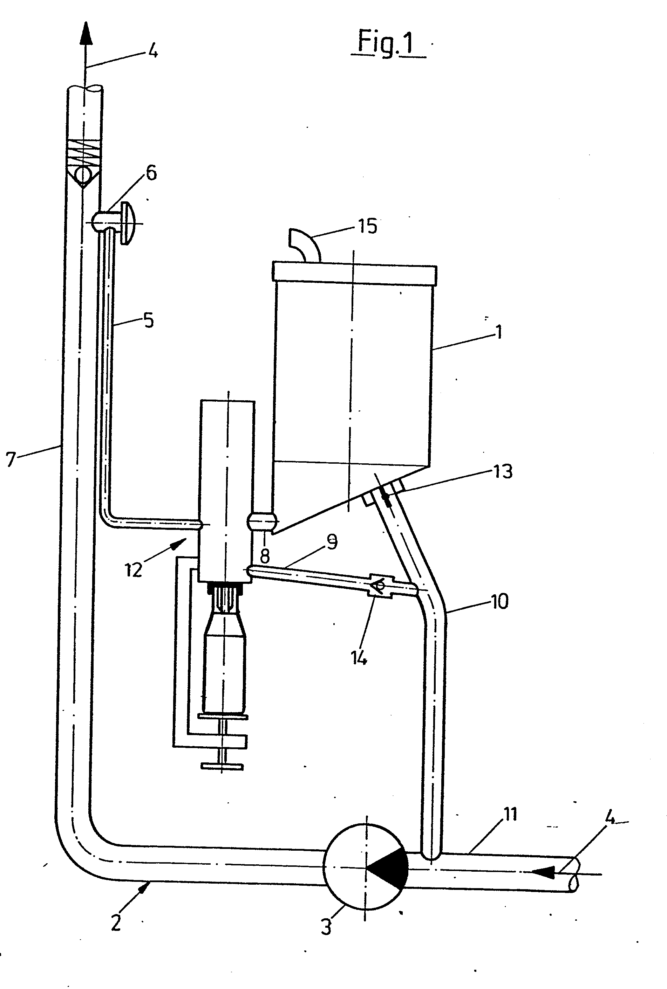

Die Abzweigung der später zur Entnahme einer Milchprobe verwendeten Teilmilchmenge erfolgt über eine Zulaufleitung 5, die bei der Ausführung nach Fig. 1 über ein Stellventil 6 an den druckseitigen, d.h. hinter der Pumpe 3 liegenden, Teil 7 der Übernahmeleitung 2 angeschlossen ist. Mit Hilfe des Stellventils 6 kann der Mengenanteil der fortlaufend abgezweigten Teilmilchmenge in Bezug auf die je Übernahmevorgang angenommene Gesamtmilchmenge verändert werden.

Die Zulaufleitung 5 kann direkt in den Zwischenbehälter 1 münden, wobei eine Einmündung im unteren Behälterbereich bevorzugt ist, da in diesem Falle die zulaufende Milch jene sich bereits im Zwischenbehälter 1 befindende Teilmilchmenge durch Strahlwirkung durchrührt und verhindert, daß sich Absetzschichtungen in der abgezweigten Teilmilchmenge bilden.

Der Zwischenbehälter 1 ist ferner über eine Rücklaufleitung, die bei dem dargestellten Beispiel gemäß Fig.1 u.2 aus den Leitungsteilen 8,9 und 10 besteht, an die Übernahmeleitung 2 angeschlossen, und zwar bei dem Beispiel gemäß Fig. 1 an deren saugseitigen Teil 11.

Der Teil 8 der Rücklaufleitung mündet in eine Probenabfüllvorrichtung 12 ein, aus der der Teil 9 der Rücklaufleitung ausmündet. Dieser Teil 9 mündet seinerseits wieder in den Teil 10 der Rücklaufleitung, der über ein Ventil 13 an den Bodenbereich des Zwischenbehälters 1 einerseits und andererseits an den saugseitigen Teil 11 der Übernahmeleitung 2 angeschlossen ist. Im Teil 9 der Rücklaufleitung ist ein Rückschlagventil 14 vorgesehen. Die Oberseite des Zwischenbehälters 1 besitzt einen Entlüftungsstutzen 15, über den der Innenraum des Zwischenbehälters 1 mit der Umgebung in Verbindung steht.

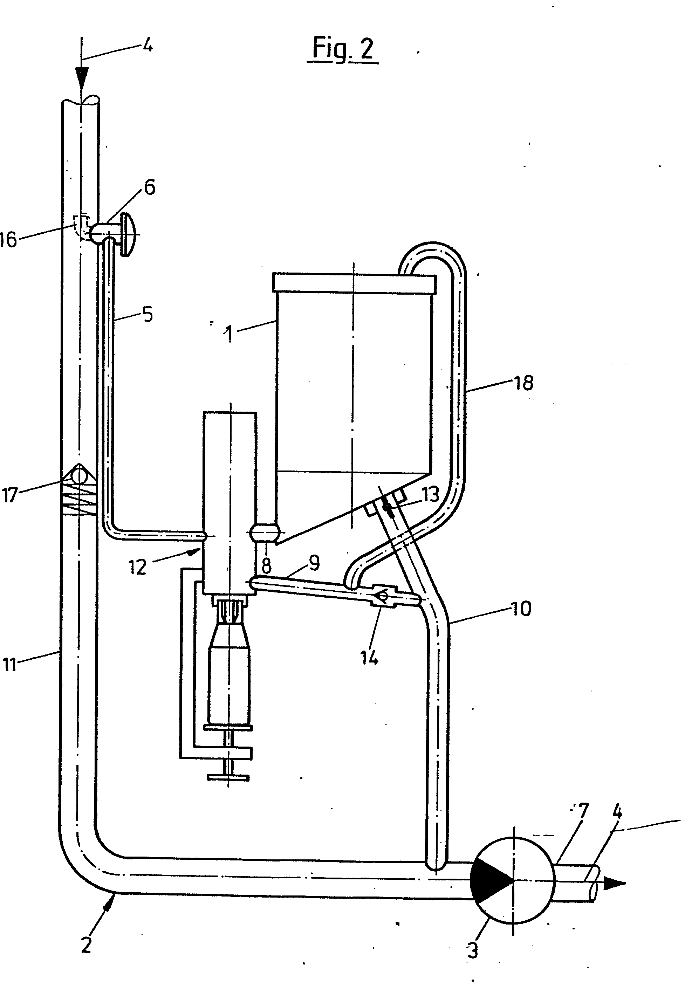

Die Ausführung gemäß Fig. 2 unterscheidet sich von der nach Fig. 1 lediglich dadurch, daß die Zulaufleitung 5 an den saugseitigen Teil 11 der Übernahmeleitung 2 angeschlossen ist und auch die Rücklaufleitung mit ihrem Teil 10 wieder in den saugseitigen Teil 11 der Übernahmeleitung 2 einmündet, jedoch in Strömungsrichtung 4 der zu übernehmenden Milch stromab der Anschlußstelle für die Zulaufleitung 5. Zwischen den Anschlußstellen der Zulaufleitung 5 und des Teils 10 der Rücklaufleitung an der Übernahmeleitung 2 besteht, wenngleich im Unterdruckbereich, ein Druckgefälle, das noch dadurch verstärkt werden kann, daß die Zulaufleitung 5 mit einem der Strömung entgegenblickenden Rohrkrümmer 16 an die Übernahmeleitung 2 angeschlossen ist. Auch ein in der Übernahmeleitung 2 zwischen den genannten Anschlußstellen angeordnetes Rückschlagventil 17 kann die zwischen den Anschlußstellen vorliegende Druckdifferenz vergrößern. Der Zwischenbehälter 1 ist als geschlossener Unterdruckbehälter ausgebildet und über eine Verbindungsleitung 18 mit dem Teil 9 der Rücklaufleitung verbunden, so daß sich der Unterdruck an der Anschlußstelle des Teils 10 der Rücklaufleitung an der Übernahmeleitung 2 über die Vorrichtungsteile 10,9, 18,1,5 und 6 fortpflanzt und eine Abzweigströmung aufgrund der schon erwähnten Druckdifferenz erzeugt.

Die in Verbindung mit den Ausführungsbeispielen nach Fig. 1 und 2 vorgesehene Probenabfüllvorrichtung 12 besteht im einzelnen aus einem Ventilgehäuse 19, das einen kreisförmigen Querschnitt haben kann und in seinem oberen Bereich an den Teil 8 der Rücklaufleitung angeschlossen ist. In seinem Boden 20 weist das Ventilgehäuse 19 eine Bodenöffnung 21 auf, unter der aufeinanderfolgend jeweils Probegefäße 22 bereitstellbar sind. In Bodenhöhe mündet aus dem Ventilgehäuse der Teil 9 der Rücklaufleitung aus, so daß das Ventilgehäuse insgesamt in die Rücklaufleitung eingeschaltet ist.

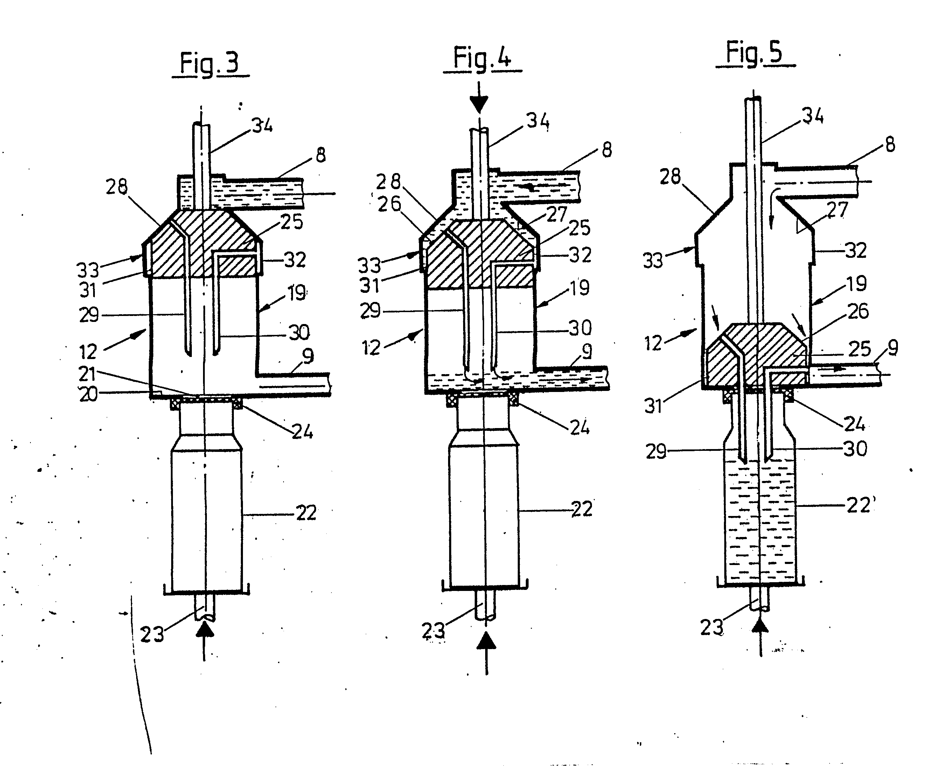

In der in den Fig. 3 bis 5 veranschaulichten Bereitstellung, in der der Probebehälter 22 mittels einer Hubvorrichtung 23 angehoben und mit einer Gummi-Verschlußkappe 24 unter Abdichtung der Bodenöffnung 21 an den Boden 20 des Ventilgehäuses 19 angedrückt ist, wird der Probebehälter 22 bis zu einer bestimmten Füllhöhe mit einer Probemilchmenge in noch nachstehend zu beschreibender Weise gefüllt, wenn der Übernahmevorgang abgeschlossen ist.

Im Ventilgehäuse 19 der Probenabfüllvorrichtung 12 ist ein Ventilkörper 25 vorgesehen, der in seiner in Fig. 3 obersten Stellung durch Dichtungswirkung den Eintritt von Milch aus dem Teil 8 der Rücklaufleitung in das Ventilgehäuse 19 verhindert. Hierzu besitzt der als Kolben ausgebildete Ventilkörper 25 oberseitig eine kegelige Ringdichtungsfläche 26, der eine entsprechende kegelige Ventilsitzfläche 27 im Deckelbereich 28 des Ventilgehäuses 19 gegenüberliegt.

Der Ventilkörper oder Kolben 25 trägt zwei durch diesen hindurchgehende, nach unten über die Kolbenunterseite vertikal vorstehende Injektionshohlnadeln 29,30, von denen die eine Injektionshohlnadel 29 mit ihrem oberen Ende aus der Ringdichtungsfläche 26 ausmündet und von denen die zweite Injektionshohlnadel 30 mit ihrem oberen Ende aus der Seitenumfangsfläche 31 des Kolbens 25 ausmündet. In seinem nach unten hin an die Ventilsitzfläche 27 angrenzenden Seitenumfangsbereich 32 weist das Ventilgehäuse 19 eine ringsumlaufende Erweiterung 33 auf, die sicherstellt, daß in der in Fig. 4 dargestellten, etwas gegenüber der Stellung nach Fig. 3 abgesenkten Zwischenstellung in den oberen Bereich des Ventilgehäuses 19 eintretende Milch die zweite Injektionshohlnadel 30 ebenso wie die erste Injektionshohlnadel 29 durchströmen kann. In dieser Zwischenstellung gemäß Fig. 4 ist die Dichtungswirkung zwischen den Flächen 26,27 aufgehoben und durch den Teil 8 der Rücklaufleitung zuströmende Milch strömt durch die beiden Injektionshohlnadeln 29,30 aus dem oberen Bereich des Ventilgehäuses in dessen unteren Bereich, wobei die Injektionshohlnadeln 29, 30 noch innerhalb des unteren Bereichs des Ventilgehäuses 19 enden. Auf diese Weise tritt eine Durchspülung des Ventilgehäuses 19 mit rücklaufender Milch aus dem Zwischenbehälter 1 ein, die dafür Sorge trägt, daß etwa noch an den Wandungen anhaftende Restmilchmengen fortgespült werden, bevor die Milchprobe genommen wird. Hierdurch ist ein hohes Maß an Verschleppungsarmut gewährleistet.

In der in Fig. 5 veranschaulichten unteren Endstellung des Ventilkörpers oder Kolbens 25 liegt der Kolben 25 auf dem Boden 20 des Ventilgehäuses 19 auf,wobei er zugleich eine Abdichtung ringsum die Bodenöffnung 21 herstellt. Die Injektionshohlnadeln 29,30 haben nach Durchstechen der Verschlußkappe 24 ihre dargestellte Lage im Innenraum des Probebehälters 22 erhalten, und Milch strömt durch die Injektionshohlnadeln 29 aus dem oberen Bereich des Ventilgehäuses in den Innenraum des Probebehälters 22. Die Injektionshohlnadel 30 ist mit ihrem oberen Ende in Verbindung mit dem Teil 9 der Rücklaufleitung, so daß in den Probebehälter 22 durch die Injektionshohlnadel 29 abgefüllte Milch zum Teil 9 der Rücklaufleitung hin abströmt, sobald der Milchpegel im Probebehälter 22 die dargestellte Ebene in Höhe der unteren Mündungsöffnung der Injektionshohlnadel 30 erreicht. Auf diese Weise ist eine Mengenbegrenzung der abgefüllten Probemilchmenge gewährleistet, während gleichzeitig die Möglichkeit besteht, auch eine Durchströmung des Innenraumes des Probebehälters 22 herbeizuführen, bis durch ein Hochfahren des Kolbens 25 in seine oberste Stellung gemäß Fig. 3 der Probenabfüllvorgang abgeschlossen wird.

Bei Beginn eines Milchübernahmevorganges befindet sich der Kolben 25 in seiner in Fig. 3 veranschaulichten Stellung, in der er als Ventilkörper einen Durchlaß von Milch durch das Ventilgehäuse und damit die Rücklaufleitung verhindert. Ist die gesamte zu übernehmende Milchmenge durch die Übernahmevorrichtung in den Milchsammelbehälter übernommen worden, so befindet sich eine anteilige Teilmilchmenge im Zwischenbehälter 1, und infolge eines Weiterlaufens der Pumpe 3 wird nun über die Zulaufleitung 5 Luft in den Zwischenbehälter 1 eingefördert, die zu einem nochmaligen Durchmischen der Teilmilchmenge im Zwischenbehälter 1 führt. Dies gilt sowohl dann, wenn die Zulaufleitung 5 direkt in den Zwischenbehälter 1 mündet, als auch dann, wenn z.B. die Zulaufleitung 5 in das in Fig. 3 bis 5 linke Ende des Teils 8 der Rücklaufleitung einmündet, was alternativ ebenso gut möglich ist.

Mittels einer Kolbenstange 34 wird nun der Kolben 25 aus seiner obersten Stellung gemäß Fig. 3 in seine Zwischenstellung gemäß Fig.4 überführt, um zum einen einen Rücklauf der Teilmilchmenge aus dem Zwischenbehälter 1 in die Übernahmeleitung 2 herbeizuführen, und um zum anderen den Teil 8 der Rücklaufleitung, das Ventilgehäuse 19 und die Injektionshohlnadel 29, 30 zu durchspülen und von etwa anhaftender Restmilch eines vorhergehenden Übernahme- und Probeentnahmevorgangs zu befreien.

Nach einer geeigneten Spüldauer wird dann der Kolben 25 in seine Probeabfüllstellung gemäß Fig. 5 überführt, in der bei gleichzeitig weiterlaufendem Milchrücklauf die gewünschte Probemenge an Milch in den Probebehälter 22 abgefüllt wird. Der Kolben 25 kann in dieser untersten Stellung gemäß Fig.5 verbleiben, bis die gesamte Teilmilchmenge aus dem Zwischenbehälter 1 in die Übernahmeleitung 2 zurückgeführt ist und nur noch die Probemenge an Milch im Probebehälter 22 verbleibt. Da dies jedoch einige Zeit in Anspruch nimmt, besteht auch die Möglichkeit, unmittelbar oder zu einem geeigneten Zeitpunkt nach Abfüllen der Probemilchmenge in den Probebehälter 22 den Kolben wieder in seine obere Stellung gemäß Fig. 3 zurückzuführen. Hierdurch wird der Milchrücklauf durch die Teile 8 und 9 der Rücklaufleitung unterbrochen, und zur vollständigen Rückführung der Teilmilchmenge aus dem Zwischenbehälter 1 kann nun das Ventil 13 geöffnet werden, wodurch durch den Teil 10 der Rücklaufleitung die restliche Teilmilchmenge aus dem Zwischenbehälter 1 in die Übernahmeleitung 2 zurückführ-bar ist. Nach Entleerung des Zwischenbehälters 1 ist dann die Vorrichtung für einen erneuten Übernahme- und Probeentnahmevorgang bereit.

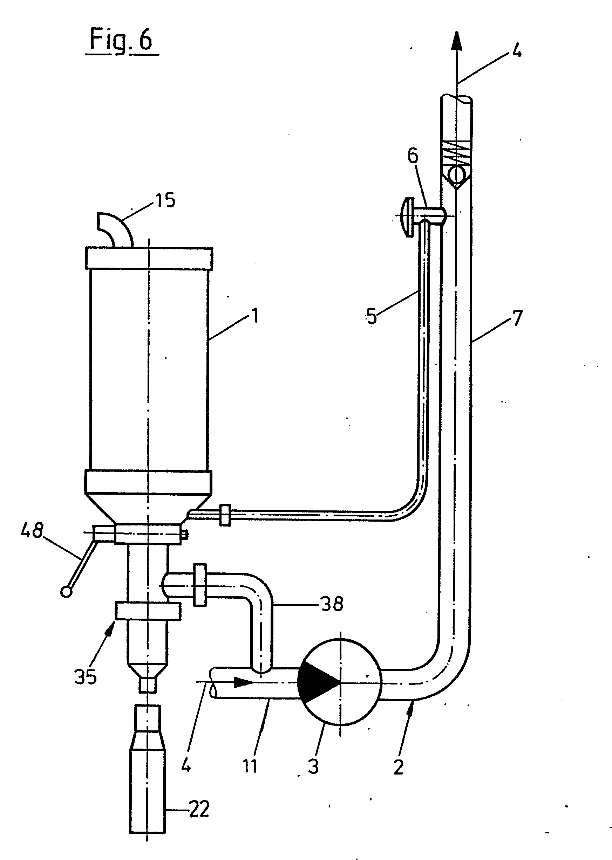

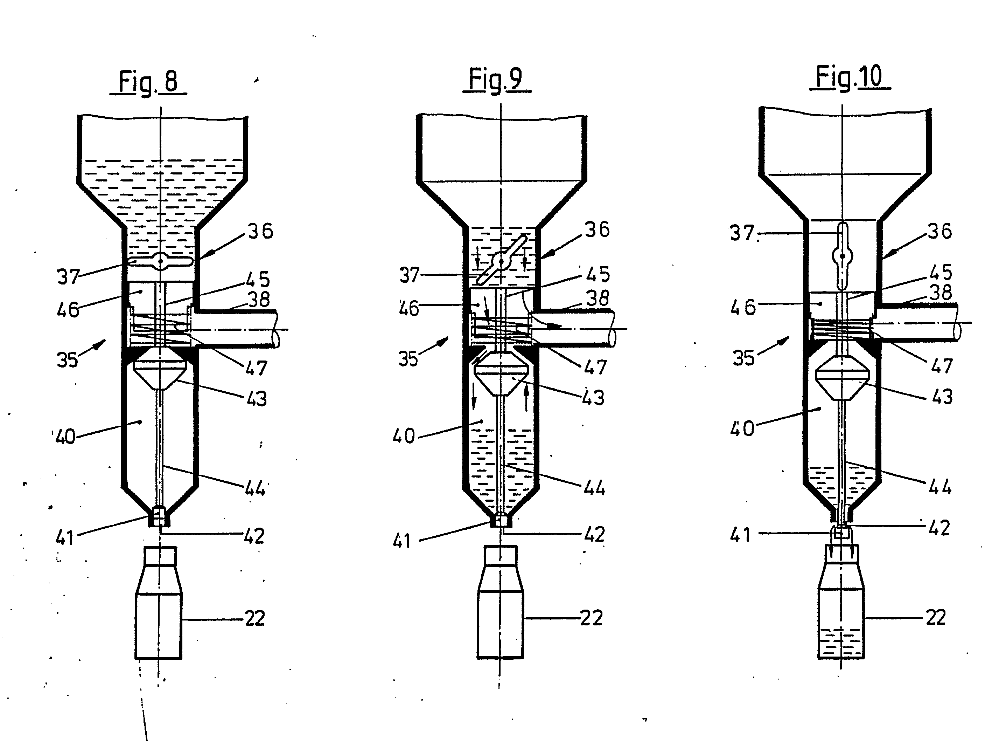

Bei den Ausführungen gemäß den Fig. 6 bis 10 ist die Probenabfüllvorrichtung 35 mit dem oberen Bereich ihres Ventilgehäuses 36 unmittelbar an den unteren Bereich des Zwischenbehälters 1 angebaut. Dabei ist im Übergangs- oder im oberen Bereich des Ventilgehäuses 36 ein erster Ventilkörper 37 angeordnet, der in seiner in Fig. 8 veranschaulichten Verschlußstellung ein Abströmen von Milch aus dem Zwischenbehälter 1 in das Ventilgehäuse 36 verhindert. Im Abstand unterhalb des ersten Ventilkörpers 37 mündet aus dem vorzugsweise im wesentlichen zylindrischen Ventilgehäuse 16 seitlich eine Rücklaufleitung 38 aus, die bei dem Beispiel gemäß Fig. 6 in den saugseitigen Teil 11 der Übernahmeleitung 2 mündet, während die Zulaufleitung 5 zum unteren Bereich des Zwischenbehälters 1 aus dem druckseitigen Teil 7 der Übernahmeleitung 2 ausmündet.

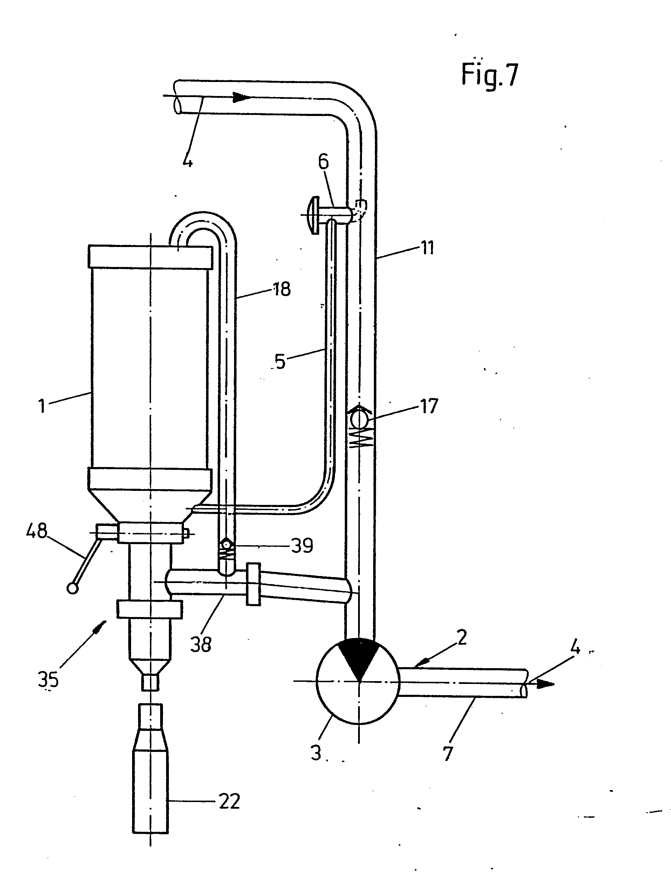

Bei der Ausführung gemäß Fig. 7 mündet die Rücklaufleitung 38 in den saugseitigen Teil 11 der Übernahmeleitung 2, und zwar in Richtung des Pfeiles 4 der bei der Übernahme strömenden Milch stromab der Anschlußstelle der Zulaufleitung 5 an die Übernahmeleitung 2. Hierbei ist der Zwischenbehälter 1 im übrigen wiederum als geschlossener Unterdruckbehälter ausgebildet und steht über eine Verbindungsleitung 18, die aus dem oberen Bereich des Zwischenbehälters 1 ausmündet, mit der Rücklaufleitung 38 in Verbindung, wobei in der Verbindungsleitung 18 ein Rückschlagventil 39 eingebaut sein kann.

Im Bereich unterhalb der Anschlußstelle der Rücklaufleitung 38 bildet das Ventilgehäuse 36 eine Probemilchkammer 40, die eine untere, mittels eines zweiten Ventilkörpers 41 absperr- und freigebbare Auslauföffnung 42 besitzt. Die Probemilchkammer 40 ist vom darüber liegenden Bereich des Ventilgehäuses 36 durch einen dritten Ventilkörper 43 abtrennbar, der mit einem Ventilsitz 44 unmittelbar unterhalb der Ausmündung der Rücklaufleitung 38 im Gehäuse 36 zusammenwirkt. Der zweite Ventilkörper 41 ist mit dem dritten Ventilkörper 43 durch eine Verbindungsstange 44' gekuppelt, und der dritte Ventilkörper 43 trägt eine obere Kolbenstange 45, auf der ein im Querschnitt etwa kreuzförmiges Betätigungsglied 46 befestigt ist. Zwischen diesem und dem Ventilsitz 44 ist eine Feder 47 angeordnet, welche bestrebt ist, die Teile 41,43,44,45 und 46 nach oben, d.h. in ihre in Fig. 8 veranschaulichte Schließstellung zu drücken.

Bei der Stellung der Teile der Probenabfüllvorrichtung 35, wie sie in Fig. 8 veranschaulicht ist, wird der Milchübernahmevorgang durchgeführt und die sich im Zwischenbehälter 1 ansammelnde Teilmilchmenge abgezweigt. Ein Milchrücklauf findet hierbei nicht statt. Ist nun der Milchübernahmevorgang abgeschlossen, erfolgt durch über die Zulaufleitung 5 in den Zwischenbehälter 1 einströ-mende Luft bei weiterlaufender Pumpe 3 ein fortgesetztes Durchmischen der Teilmilchmenge im Zwischenbehälter 1 statt, wonach zu einem geeigneten Zeitpunkt nunmehr der erste Ventilkörper 37 aus seiner Schließstellung in Fig. 8 in eine Zwischenstellung gemäß Fig. 9 bewegt wird, z.B. mit Hilfe des Handhebels 48. Der als Schwenkklappe ausgebildete erste Ventilkörper 37 betätigt bei seiner Bewegung in die Zwischenstellung gemäß Fig. 9 über die Teile 45,46 und entgegen der Wirkung der Feder 47 den dritten Ventilkörper 43 im Sinne eines Abhebens von seinem Ventilsitz 44. Bei dabei zunächst noch geschlossener Auslauföffnung 42 gelangt Milch aus dem Zwischenbehälter 1 vorbei am ersten Ventilkörper 37 zum einen in die Rücklaufleitung 38 und zum anderen in die Probemilchkammer 40, die durchspült wird und aus der die Milch wieder zurück und in ebenfalls die Rücklaufleitung 38 gelangt. Das kreuzförmige Betätigungsglied 46 bildet dabei einen Leitkörper, der die gewünschte Schleifenströmung in der Probemilchkammer 40 sichert. In der Stellung der Teile gemäß Fig. 9 tritt auch hier wieder eine vollständige Durchspülung der Probenabfüllvorrichtung 35 ein mit der Folge, daß vor einer Abfüllung einer Probemilchmenge in den Probebehälter 22 etwa anhaftende Restmilch aus einem vorhergehenden Übernahmevorgang entfernt wird. Nachdem die gesamte Teilmilchmenge über die Rücklaufleitung 38 zurückgeführt ist, bleibt lediglich die Probemilchkammer 40 mit Probemilch eine durch ihre die Kammerabmessungen bestimmten Menge gefüllt, und es kann nun eine Abfüllung dieser Probemilchmenge in den unter der Auslauföffnung 42 bereitgestellten Probebehälter 22 erfolgen. Dies geschieht durch vollständiges Öffnen des ersten Ventilkörpers 37, der in dieser Stellung in Fig. 10 veranschaulicht ist und über die Teile 46,45,43,44 nunmehr auch den zweiten Ventilkörper 41 so weit absenkt, daß dieser die Auslaßöffnung 42 für einen Schwerkraftablauf der Probemilch aus der Probemilchkammer 40 in den Probebehälter 22 freigibt.

Nach erfolgter Abfüllung der Milchprobe wird nun der erste Ventilkörper 37 wieder in seine Schließstellung gemäß Fig. 1 gebracht, wodurch die Ventilkörper 41,43 ebenfalls durch die Wirkung der Feder 37 in ihre Schließstellung zurückkehren. Die Vorrichtung ist nunmehr für die Durchführung eines erneuten Übernahme- und Probeentnahmevorganges bereit.

Die Ventilkörper 37,41,43 bilden gemeinschaftlich gewissermaßen ein Mehrstufenventil mit einer vorgegebenen Reihenfolge ihrer Betätigung, wobei die Betätigung allein des ersten Ventilkörpers 37 zunächst lediglich den Milchrücklauf öffnet, dann die Probemilchkammer 40 oberseitig öffnet und schließlich den Ventilkörper 41 aus seiner Schließlage herausbewegt, damit die Probemilchmenge ablaufen
kann.

- 12 -

Ansprüche:

1.    Vorrichtung zur Entnahme von Milchproben aus einer bei der Übernahme von Milch in einen Milchsammelbehälter aus der Übernahmeleitung einer Übernahmevorrichtung abgezweigten, in einem Zwischenbehälter angesammelten Teilmilchmenge, wobei der Zwischenbehälter über eine Zulaufleitung und über eine Rücklaufleitung an die Übernahmeleitung angeschlossen sowie mit einer ventilgesteuerten Probenabfüllvorrichtung versehen ist, unter deren Auslauf Probebehälter bereitstellbar sind, dadurch gekennzeichnet, daß die Probenabfüllvorrichtung (12;35) ein in die Rücklaufleitung (8,9,10;38) eingeschaltetes Ventilgehäuse (19;36) und darin einen Ventilkörper (25;37) umfaßt, der in einer ersten Stellung einen Durchlaß durch das Ventilgehäuse und damit die Rücklaufleitung sperrt, in einer zweiten Stellung einen Durchlaß im Ventilgehäuse für einen dieses durchspülenden Milchrücklauf durch die Rücklaufleitung öffnet und in einer dritten Stellung zusätzlich einen Probemilchauslauf in den bereitgestellten Probebehälter (22) freigibt.

2.    Vorrichtung nach Anspruch 1, dadurch gekennzeichnet, daß das Ventilgehäuse (19) der Probenabfüllvorrichtung (12) oberseitig über einen Teil (8) der Rücklaufleitung an den Zwischenbehälter (1) angeschlossen, unterseitig mit einer durch eine Verschlußkappe (24) eines bereitgestellten Probebehälters (22) verschließbaren Bodenöffnung (21) versehen und seitlich in Bodenhöhe mit einem weiteren Teil (9) der Rücklaufleitung verbunden ist sowie einen im Ventilgehäuse auf und ab verschiebbaren Kolben (25) als Ventilkörper umfaßt, der zwei diesen durchquerende, nach unten über die Kolbenunterseite vorstehende, durch die Bodenöffnung und die Verschlußkappe eines Probebehälters hindurch in diesen einführbare Injektionshohlnadeln (29,30) trägt, wobei die Injektionshohlnadeln in der obersten Kolbenstellung gegen ein Durchströmen von Milch gesperrt sind, in einer Zwischenstellung des Kolbens eine Durchlaßverbindung zwischen dem oberen und dem unteren Bereich des Ventilgehäuses herstellen und in der untersten Stellung des Kolbens den Innenraum des Probenbehälters mit dem oberen Bereich des Ventilgehäuses einerseits und mit der Rücklaufleitung andererseits verbinden.

3.      Vorrichtung nach Anspruch 2, dadurch gekennzeichnet, daß der Kolben (25) oberseitig eine kegelige Ringdichtungsfläche (26) aufweist, der eine entsprechende kegelige Ventilsitzfläche (27) im Deckelbereich (28) des Ventilgehäuses (19) gegenüberliegt, und daß das obere Ende der einen Injektionshohlnadel (29) aus der Ringdichtungsfläche und das der zweiten Injektionshohlnadel (30) aus einer Seitenumfangsfläche (31) des Kolbens (25) ausmündet.

4.      Vorrichtung nach Anspruch 2 oder 3, dadurch gekennzeichnet, daß das Ventilgehäuse (19) in einem nach unten hin an die Ventilsitzfläche (27) angrenzenden Seitenumfangsbereich (32) eine Erweiterung (33) aufweist, die der Seitenumfangsfläche (31) des Kolbens (25) im Abstand gegenüberliegt.

5.      Vorrichtung nach Anspruch 4, dadurch gekennzeichnet, daß in der Zwischenstellung des Kolbens (25) die Erweiterung (33) eine Verbindung zwischen dem oberen Bereich des Ventilgehäuses (19) und dem oberen Ende der zweiten Injektionshohlnadel (30) herstellt und die unteren Enden der beiden
Injektionshohlnadeln (29,30) oberhalb der Bodenöffnung (21)
des Ventilgehäuses (19) gelegen sind.

6.      Vorrichtung nach einem oder mehreren der Ansprüche
2 bis 5, dadurch gekennzeichnet, daß der in Bodenhöhe an das
Ventilgehäuse (19) angeschlossene Teil. (9) der Rücklaufleitung an einen weiteren Teil (10) der Rücklaufleitung angeschlossen ist, der über ein Steuerventil (13) einerseits
mit dem Bodenbereich des Zwischenbehälters (1) und andererseits mit der Übernahmeleitung (2) verbunden ist.

7.      Vorrichtung nach Anspruch 1, dadurch gekennzeichnet,
daß das Ventilgehäuse (36) der Probenabfüllvorrichtung (35)
mit seinem oberen Bereich unmittelbar an den unteren Bereich
des Zwischenbehälters (1) angebaut und im oberen Bereich
des Ventilgehäuses ein erster Ventilkörper (37) angeordnet
ist, im Abstand unterhalb des ersten Ventilkörpers die
Rücklaufleitung (38) seitlich aus dem Ventilgehäuse ausmündet und unterhalb der Ausmündung der Rücklaufleitung eine
Probemilchkammer (40) im Ventilgehäuse ausgebildet ist, die
eine untere, mittels eines zweiten Ventilkörpers (41) absperr- und freigebbare Auslauföffnung (42) aufweist.

8.      Vorrichtung nach Anspruch 7, dadurch gekennzeichnet,
daß die Probemilchkammer (40) vom darüberliegenden Bereich
des Ventilgehäuses (36) durch einen dritten Ventilkörper
(43) abtrennbar ist, der unmittelbar unterhalb der Ausmündung der Rücklaufleitung (38) mit einem Ventilsitz (44)
im Ventilgehäuse zusammenwirkt.

9.      Vorrichtung nach Anspruch 8, dadurch gekennzeichnet,
daß der zweite und der dritte Ventilkörper (41,43) durch
ein Verbindungsglied (44) untereinander gekuppelt und durch
den ersten Ventilkörper (37) betätigbar sind.

10.    Vorrichtung nach Anspruch 9, dadurch gekennzeichnet, daß die drei Ventilkörper (37,41,43) ein Mehrstufenventil mit abgestuft aufeinanderfolgenden Öffnungsfunktionen bilden.

11.    Vorrichtung nach einem oder mehreren der Ansprüche 1 bis 10, dadurch gekennzeichnet, daß die Zulaufleitung (5) zum Zwischenbehälter (1) aus dem druckseitigen Teil (7) der Übernahmeleitung (2) ausmündet, der Zwischenbehälter (1) zur Umgebung hin entlüftet ist und die Rücklaufleitung (8,9,10;37) in den saugseitigen Teil (11) der Übernahmeleitung einmündet.

12.    Vorrichtung nach einem oder mehreren der Ansprüche 1 bis 10, dadurch gekennzeichnet, daß die Zulaufleitung (5) aus dem saugseitigen Teil (11) der Übernahmeleitung (2) ausmündet, der Zwischenbehälter (1) als zur Umgebung hin abgeschlossener Unterdruckbehälter ausgebildet und über eine Verbindungsleitung (18) an die Rücklaufleitung (8,9,10;38) angeschlossen ist, und die Rücklaufleitung in Richtung (4) der in der Übernahmeleitung (2) strömenden Milch stromab der Anschlußstelle der Zulaufleitung in den saugseitigen Teil (11) der Übernahmeleitung mündet.

0098966

Fig.1

Fig. 2

Fig.3    Fig.4    Fig.5

## Fig. 6

Fig.7

Fig. 8    Fig. 9    Fig. 10

9/9

9968966